# EUROPEAN PATENT SPECIFICATION

(11) **EP 3 762 403 B1**
(45) Date of publication and mention of the grant of the patent: **01.05.2024**
(21) Application number: 19717193.7
(22) Date of filing: 05.03.2019
(51) Int. Cl.: C07K 14/435, A61K 38/00

(54) **A NEUROTROPHIC PEPTIDE FOR THE THERAPEUTIC TREATMENT OF NEURODEGENERATIVE AND/OR INFLAMMATORY DISEASES**
NEUROTROPHES PEPTID ZUR THERAPEUTISCHEN BEHANDLUNG VON NEURODEGENERATIVEN UND/ODER ENTZÜNDLICHEN ERKRANKUNGEN
PEPTIDE NEUROTROPHIQUE POUR LE TRAITEMENT THÉRAPEUTIQUE DE MALADIES NEURODÉGÉNÉRATIVES ET/OU INFLAMMATOIRES

(30) Priority: 05.03.2018 IT 201800003279
(43) Date of publication of application: 13.01.2021
(73) Proprietor: Consiglio Nazionale Delle Ricerche, 00185 Roma (IT)
(72) Inventor: MANNI, Luigi, 00078 Monte Porzio Catone (Roma) (IT); SOLIGO, Marzia, 00073 Castel Gandolfo (Roma) (IT); BRACCI-LAUDIERO, Luisa, 00193 Roma (IT)
(74) Representative: Rimini, Rebecca
(86) International application number: PCT/IB2019/051753
(87) International publication number: WO 2019/171261

(56) References cited:
- WO-A2-2008/097927
- WO-A2-2013/151666
- WO-A2-2017/191274
- DATABASE Geneseq [Online] NCBI; 12 March 2015 (2015-03-12), "PREDICTED: Homo sapiens nerve growth factor (beta polypeptide) (NGF), transcript variant X1, mRNA", XP002785282, retrieved from EBI Database accession no. XM_011541518
- DATABASE EMBL [Online] 29 August 2018 (2018-08-29), "Homo sapiens pro-nerve growth factor long variant (NGF) mRNA, complete cds, alternatively spliced.", XP002785283, retrieved from EBI accession no. EM_NEW:MH358394 Database accession no. MH358394
- ULLRICH A ET AL: "HUMAN BETA-NERVE GROWTH FACTOR GENE SEQUENCE HIGHLY HOMOLOGOUS TO THAT OF MOUSE", NATURE, MACMILLAN JOURNALS LTD, LONDON, GB, vol. 303, 30 June 1983 (1983-06-30), pages 821-825, XP002947062, ISSN: 0028-0836, DOI: 10.1038/303821A0 cited in the application
- P. C. PAGADALA ET AL: "Construction of a mutated pro-nerve growth factor resistant to degradation and suitable for biophysical and cellular utilization", PROCEEDINGS OF THE NATIONAL ACADEMY OF SCIENCES OF THE UNITED STATES OF AMERICA, vol. 103, no. 47, 21 November 2006 (2006-11-21), pages 17939-17943, XP055290825, ISSN: 0027-8424, DOI: 10.1073/pnas.0604139103

## Description

The present invention falls within the field of therapeutic treatments of acute or chronic neurodegenerative.

Neurodegenerative diseases are diseases of extreme clinical and epidemiological relevance, with a considerable socio-economic impact, but at the same time characterized by the lack of effective therapeutic treatments.

Alzheimer's Disease, Parkinson's disease and Huntington's Chorea are among the most representative examples of chronic neurodegenerative forms that tend to arise and worsen with age. Since they are associated with progressive structural and functional alteration of the central nervous system (CNS), these diseases involve a gradual and progressively disabling impairment of the patient's mental and cognitive faculties, as well as his/her motor functions.

Neurodegenerative processes also occur following a vascular or traumatic, acute brain damage. Among these, ischemic stroke is one of the world's main causes of disability and death, and the incidence of this disease also increases with age. In most cases, stroke is caused by thrombosis of the main cerebral arteries. The ischemic event shares several pathophysiological mechanisms with traumatic brain injury (TBI) that statistics indicate as the leading cause of death among individuals under the age of 44, and both of these pathological phenomena triggered by acute episodes lead to severe, both motor and cognitive, debilitating deficiencies.

In recent years, an increasing amount of experimental evidence has demonstrated that acute and chronic neurodegenerative diseases, although differing in symptoms, initial underlying causes and brain areas affected by the damage, exhibit common pathogenetic mechanisms which together contribute to cause the high degree of neuronal death primarily characterizing the onset and progression of these diseases.

Another group of diseases with high prevalence and significant impact on patients' living conditions are chronic inflammatory diseases. Although they include very different diseases, including for example rheumatoid arthritis and systemic lupus erythematosus, these disorders are anyhow related to a malfunction of the immune system and share a chronic inflammatory condition resulting in major injuries to different organs. Patients suffering from these types of diseases are usually constantly monitored for their condition, often throughout their lifetime, and for possible complications associated with chronic exposure to pharmaceutical therapies they undergo.

The Nerve Growth Factor (NGF) was the first neurotrophin to be identified in the 1950s and its biological effects on neuronal proliferation and survival and nerve fibre growth are well known. NGF, like other secreted proteins, is synthesized from a precursor protein, the pronerve growth factor (proNGF), which can be processed into mature neurotrophin inside the cell, or alternatively can be secreted and subject to proteolytic events by extracellular proteases. The proNGF molecule has its own biological activity and several studies have shown a dualism between the action of this precursor and that of the mature neurotrophin (Fahnestock, M., et al. ProNGF: a neurotrophic or an apoptotic molecule? Prog Brain Res, 2004. 146: p. 101-10). In fact, proNGF can exert a neurotoxic effect, related to the activation of the p75-sortilin receptor complex, and a pro-apoptotic effect, the latter being responsible for cell death under physiological conditions, for example during development, as well as in the context of neurodegenerative diseases. In contrast, NGF is also attributed a neurotrophic role, being responsible for the differentiation and maintenance of the phenotypic characteristics of cholinergic neurons in the central nervous system and catecholaminergic neurons in the peripheral nervous system. The hypothesis was also suggested that proNGF activity might be mediated by the activation of the neurotrophic TrkA receptor. So far, only one proNGF precursor, designated as proNGF-B, has been identified in humans by cloning the corresponding encoding gene using a mouse beta-NGF cDNA (Ullrich A et al.; Human beta-nerve growth factor gene sequence highly homologous to that of mouse Nature. 1983 303(5920):821-5). Mature NGF factor originates from the carboxy-terminal portion of this protein, which is 221 amino acids long. Hempstead B.L. discloses that said proNGF precursor is a distinct, biologically active ligand than mature neurotrophin and induces opposing actions on neurons by promoting apoptosis and neurotoxicity (Hempstead B.L. Handb Exp Pharmacol 2014, 220:17-32).

The presence in humans of another proNGF variant was also assumed on the basis of automatic computational analyses of the human genome, whose predicted coding sequence is accessible from the NCBI database at the following link: https://www.ncbi.nlm.nih.gov/nuccore/XM_011541518. Under said NCBI entry a beta-NGF isoform X2 is disclosed along with a mRNA of 1263 base pairs (bp) encoding said isoform.

WO2017/191274 describes an amino acid sequence of an NGF peptide isoform designated as SEQ ID NO. 7460, which is 296 residues in length and is defined with reference to accession number XP_011539820 in the NCBI database (https://www.ncbi.nlm.nih.gov/protein/XP_01153982).

However, the aforementioned variants have never been isolated or even experimentally characterized, and are predicted on the basis of computational analyses.

WO2013/151666 discloses polynucleotides encoding one or more polypeptides associated with human diseases, more particularly modified mRNA molecules. Among said polynucleotides, a nucleotide sequence of 1047 bp in length is disclosed, which is referred as SEQ ID NO. 554. However, WO2013/151666 does not provide any therapeutic indication in connection with the polynucleotide of SEQ ID NO. 554 or the encoded peptide.

WO2008/097927 discloses the therapeutic use of various peptides, including the neurotrophin NGF, for the treatment of neurodegenerative and inflammatory diseases.

In recent years, the use of the NGF peptide has been examined as a therapeutic opportunity for the treatment of neurodegenerative diseases, based on the neurotrophic and neurotropic potential of this peptide. Currently, clinical trials are underway to verify the potential therapeutic application of the NGF peptide in the ophthalmological field, whose therapeutic efficacy has already been approved for the treatment of neurotrophic keratitis. Recently, the NGF peptide was also used in the treatment of a severe clinical case of brain trauma, by intranasal administration (Chiaretti, A., et al., 2017. Intranasal Nerve Growth Factor administration improves cerebral functions in a child with severe traumatic brain injury: A case report. Brain Inj 31, 1538-1547). However, the therapeutic use of this neurotrophin suffers from some major limitations, which on the one hand derive from the hyperalgesic effect of the NGF peptide and its susceptibility to degradation by extracellular matrix proteases, and on the other hand from its potential dangerous pro-tumour activity.

In this context, therefore, the dramatic need arises for the development of therapeutic strategies, which, while having the beneficial effects from the administration of the NGF peptide, do not have the aforementioned drawbacks.

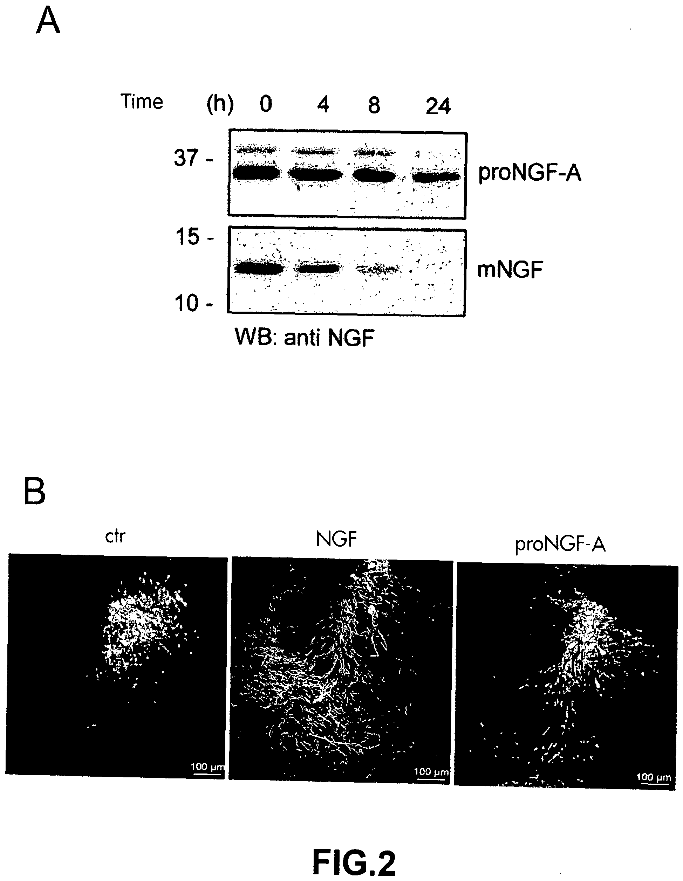

This need has now been met by the present inventors, who for the first time isolated a new variant of the human proNGF peptide and surprisingly found that said human proNGF variant exhibited neuroprotective and anti-inflammatory activities. As shown in Figure 2 and in the following experimental examples, comparative studies carried out by the present inventors have also shown that the new human proNGF variant has a significantly higher resistance to the action of extracellular proteases than the mature NGF factor. In addition, in *in vivo* models, the new proNGF variant was found to act on the central regulation of the activation of the sympathetic nervous system to a lesser extent compared to the NGF peptide. As is widely known, the sympathetic system plays a primary role in mediating physiological reactions to stress and panic, as well as in the perception of pain and in the regulation of the immune and inflammatory response. More particularly, the examination of cerebral noradrenergic nuclei, in particular of the locus coeruleus, in rat brain after administration of the new variant of the human proNGF peptide revealed the presence of a significantly lesser amount of arborization phenomena, the so-called "sprouting", by the noradrenergic fibres compared to animals treated with the mature NGF factor, thus indicating the onset of a minor response to stress and pain stimuli, as well as a more appropriate modulation of peripheral inflammation.

Therefore, one object of the present invention is an isolated peptide, hereinafter designated as human proNGF A isoform (hproNGF-A), which consists of the amino acid sequence SEQ ID NO. 1, for use in the therapeutic treatment of a neurodegenerative and/or neurotraumaticdisease.

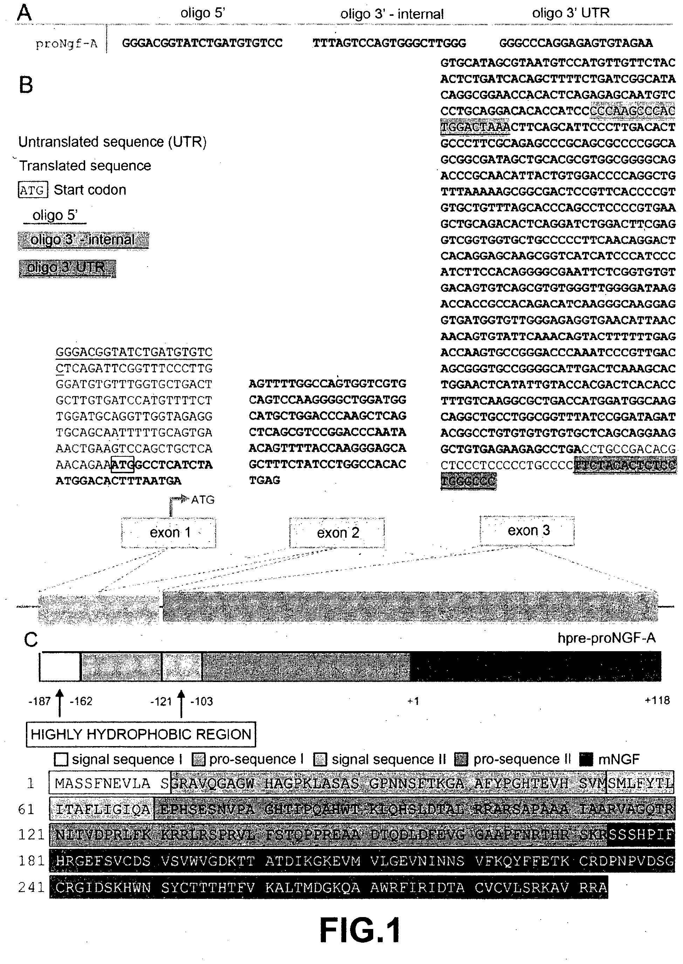

The hproNGF-A peptide for use according to the invention is characterised in that it has a molecular weight of 32-34 kDa, high resistance to degradation by the extracellular proteases plasmin and matrix metalloproteases (MMP), a length of 296 amino acids and a theoretical isoelectric point of about 9.5. Unlike the already known human hproNGF-B variant (accession number NM_002506.2 in the NCBI database), the peptide for use according to the invention contains an additional 55-amino acid, highly hydrophobic polypeptide fragment in the amino-terminal region (Figure 1C).

As will be described in detail in the following experimental section, the present inventors succeeded in identifying the peptide of the invention by isolating the corresponding nucleic acid encoding sequence from human DNA samples. In particular, the approach followed was based on the use of an *in silico* DNA sequence, predicted through computational analysis of genomic DNA, as a template for the design of a pair of amplification primers. Said primer pair was then used in a PCR reaction carried out on human cDNA samples, which allowed the transcript corresponding to the new isoform of the human proNGF peptide to be amplified and isolated for the first time. Subsequently, the full-length sequence of the cDNA molecule encoding the peptide of the invention was isolated from human brain and peripheral tissues and sequenced.

Therefore, a further object of the invention is a peptide for use as previously defined, which is encoded by nucleic acid sequence SEQ ID NO. 3.

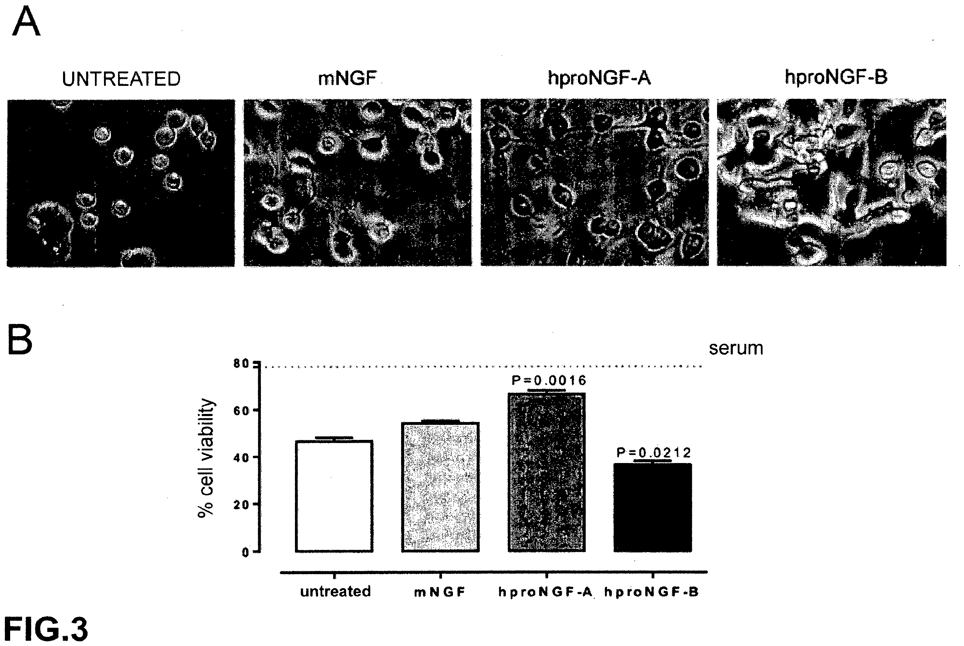

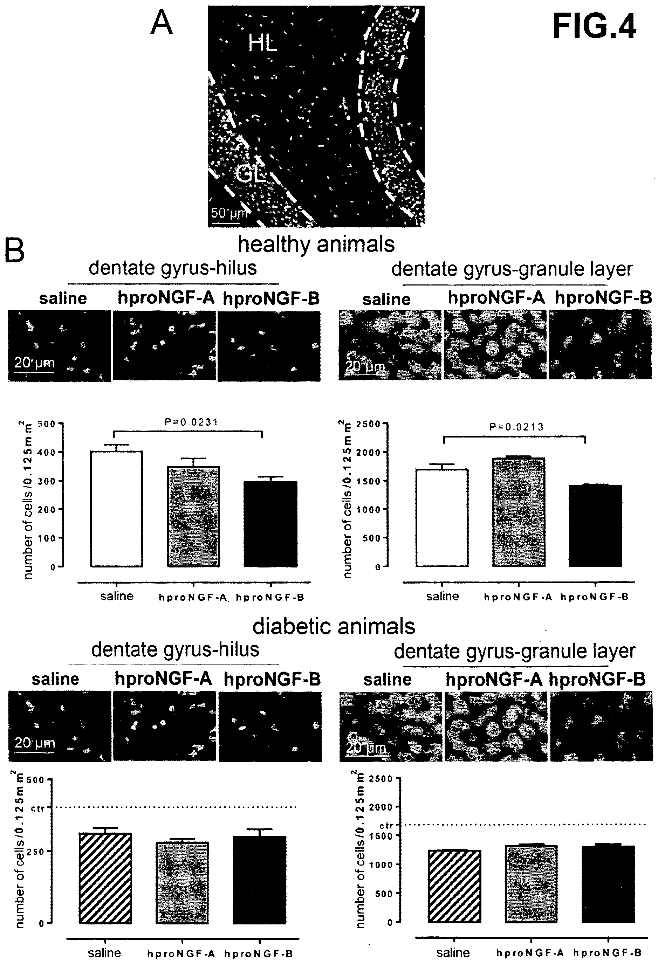

As will be explained in more detail in the following experimental section, the present inventors, through studies carried out both *in vitro* (as shown in Figure 3) and *in vivo* (as shown in Figures 4-6), have shown that the peptide for use according to the invention has the ability to promote the survival of cells responsive to NGF and their differentiation into neuronal phenotypes.

The results shown in Figures 4-6 also demonstrate that, following intranasal administration of the peptide for use according to the invention in rats suffering from diabetic encephalopathy, a known animal model of neurodegeneration, it has been possible to detect the recovery of neurophysiological functions of specific areas of the brain, the hippocampus in particular, for example measured as long-term potentiation (LTP) and related to synaptic plasticity phenomena and cognitive functions, as well as the stimulation of the neurogenesis process in the hippocampal dentate gyrus.

In addition to the protective, neuroproliferative and neuro-differentiating effects described above, the peptide for use according to the invention is also capable of counteracting inflammatory phenomena, since the use thereof induces a significant decrease in the production of pro-inflammatory cytokines in synoviocytes collected from patients with rheumatoid arthritis. The results of comparative studies carried out by the present inventors, in which the already known human hproNGF-B variant was shown instead to have neurotoxic and pro-inflammatory activity (as shown in Figures 4-6), further support what has been mentioned above.

Without wishing to be bound by theory, the present inventors have shown that the opposite biological activities exerted by the peptide for use according to the invention and the proNGF-B of the prior art could be due to activation of different cellular receptors, in particular p75 pan-neurotrophin receptor (p75NTR) by the peptide for use according to the invention, and the pro-apoptotic p75NTR-sortilin receptor complex by proNGF-B. Both the human proNGF variants are instead able to bind the TrkA receptor.

Therefore, the peptide according to the invention is particularly suitable to be used in the therapeutic treatment of a neurodegenerative and/or neuro-traumatic disease.

In the context of pathological events triggered by an acute, traumatic or vascular brain insult, mention can be made, for example, without limitation, of: (ischemic and hemorrhagic) stroke, traumatic brain injury, intracranial hypertension, cerebral edema, (perinatal, pediatric or adult) hypoxia/ischemia, hypoxia/ischemia caused by cardiac arrest, drowning or hypothermia.

With reference to chronic neurodegenerative disease patterns, mention can be made, for example, without limitation, of: Alzheimer's disease, Parkinson's disease, Huntington's

Chorea, amyotrophic lateral sclerosis (ALS), progressive supranuclear palsy, frontotemporal dementia, Lewy body dementia, Creutzfeldt-Jakob disease (CJD), Gerstmann-Sträussler-Scheinker disease (GSS).

In a preferred embodiment, the peptide for use according to the invention is particularly suitable for the therapeutic treatment of a disease selected from the group consisting of (ischemic and hemorrhagic) stroke, traumatic brain injury, intracranial hypertension, cerebral edema, (perinatal, pediatric or adult) hypoxia/ischemia, hypoxia/ischemia caused by cardiac arrest, drowning or hypothermia, Huntington's Chorea, amyotrophic lateral sclerosis (ALS), progressive supranuclear palsy, frontotemporal dementia, Creutzfeldt-Jakob disease (CJD), and Gerstmann-Sträussler-Scheinker disease (GSS).

A further object of the present invention is an expression vector for use as defined above, comprising a nucleic acid sequence encoding the peptide according to appended claim 1, preferably the nucleic acid sequence SEQ ID NO: 3 or SEQ ID NO: 2, and optionally further comprising a promoter sequence and a polyadenylation signal sequence, as well as a host cell for use as defined above, comprising the above expression vector.

Recombinant expression vectors for use in the manufacture of peptides or proteins are known and described in the state of the art, therefore the selection and use thereof are well within the skills of those of average skill in the art. Such vectors can be prokaryotic or eukaryotic vectors. By way of non-limiting example, eukaryotic vectors for expression in insect cells such as pBacPAK8, pBacPAK9, pAcG2T, pAcHLT A, pAcHLT B, pAcHLT C, pAcGHLT A, pAcGHLT B, pAcGHLT C, pAcSG2, pBAC-1, pFastBac1, pFastBacHT, pFastBac-Dual, pAcP(+)IE1, pAcP(-)IE1, pAcUW31, pBAC-1, pBAC-2cp, pBAC-3, pBAC4x-1, pBAC-7, pBAC-8, pBAC-9, pBAC-10, pBacPAK8, pBacPAK9, pBACsurf-1, pBlueBac4.5, pBlueBacHis2, pMbac, pMelBac, pPbac, pTriEx-1, pVL1392, pVL1393, are mentioned. Alternatively, the vector according to the invention may be suitable for expression in mammalian cells, for example pcDNA3, pcDNA3.1(-),pcDNA3.1(-)_myc-His A, pcDNA3.1(-)_myc-His B, pcDNA3.1(-)_myc-His C, pcDNA3.1(+), pcDNA3.1(+)_myc-His A, pcDNA3.1(+)_myc-His B, pcDNA3.1(+)_myc-His C, pcDNA3.1/Hygro(-), pcDNA3.1/Hygro(+), pcDNA3.1/V5-His A, pcDNA3.1/Zeo (-), pcDNA3.1/Zeo (+), pcDNA3.1+C-6His, pcDNA3.1+C-DYK, pcDNA3.1+C-DYK-P2A, pcDNA3.1+C-HA, pcDNA3.1+C-Myc, pcDNA3.1+N-6His, pcDNA3.1+N-DYK-P2A, pcDNA3.1+N-GST, pcDNA3.1+N-HA, pcDNA3.1+N-Myc, pcDNA3.1-C-eGFP, pcDNA3.1-N-eGFP, pcDNA3.1-P2A, pcDNA3.1-P2A-eGFP, pcDNA5/FRT, pcDNA5-TOpcDNA6/V5-His A, pCI-Neo, pCMV-3Tag-1°, pCMV-3Tag-1a-P2A, pCMV-3Tag-2°, pCMV-3Tag-3°, pCMV-3Tag-3a-P2ApCMV-3Tag-4°, pEGFP-N1, pGen2.1, pGL3-Basic, pGL4.10[luc2], pGL4.14[luc2/Hygro], pGL4.17, pHLSec). Alternatively, the vector for use according to the invention may be suitable for expression in yeast cells, for example pETCON, pYES2, pAO815, pGAPZ, pGAPZa, pHIL-D2, pHIL-S1, pPIC3.5K, pPIC9K, pPICZ, pPICZa, or for expression in bacterial cells, such as pBluescript II KS(-), pBluescript II KS(+), pBluescript II SK(-), pBluescript II SK(+), pBluescript SK(+), pCDFDuet-1, pCOLADuet-1, pColdII, pET-11°, pET-11b, pET-11c, pET-11d, pET-14b, pET-15b, pET-16b, pET-17b, pET-19bpET-20b(+), pET-21a(+), pET-21b(+), pET-21d(+), pET-22b(+) pET-23a(+), pET-24a(+), pET-24b(+), pET-24c, pET-24c(+), pET-24d , pET-24d(+), pET-25b(+), pET-26b(+), pET-27b(+), pET-28a(+), pET-28b(+), pET-28c(+), pET-29a(+), pET-29b(+), pET-29c(+), PET-30a(+), PET-30b(+), PET-30c(+), PET-31b(+), pET-32a(+), pET-32b(+), pET-3°, pET-3b, pET-3c, pET-3d, pET-41a(+), pET-41b(+), pET-41c(+), pET-42a(+), pET-42b(+), pET-42c(+), pET-43.1a(+), pET-43.1b(+), pET-45b(+), pET-50b(+), pET-51b(+), pET-52b(+), pET-9°, pETDuet-1, pGEX-2TK, pGEX-4T-1, pGEX-4T-1-H(RBS), pGEX-4T-1-M(RBS), pGEX-4T-2pGEX-4T-3, pGEX-5X-1, pGEX-5X-1-H(RBS), pGEX-5X-1-M(RBS), pGEX-5X-2, pGEX-5X-3, pGEX-6P-1, pGEX-6P-1-H(RBS), pGEX-6P-1-M(RBS), pGEX-6P-2, pGEX-6P-3, pGS-21°, pMAL-c4x, pMAL-c4x-1-H(RBS), pMAL-c4x-1-M(RBS), pMAL-c5E, pMAL-c5x, pMAL-p5E, pMAl-p5g, pMAl-p5x, pQE-1, PQE30, pQE32, pQE-60, pRSFDuet-1, pUC18, pUC19.

Restriction reactions, both with blunt-end technologies, for example Topo-cloning, and through methods such as TA cloning, GATEWAY Cloning Technology, In-Fusion, Ligation independent cloning (LIC), Di- o multi-cistronic cloning, GenEZ^{™} cloning, can be used to clone the nucleotide sequence encoding the peptide for use according to the invention in a suitable vector as described above. In addition, tag sequences can be employed at the N- and/or C-terminus, as well as specific promoter sequences and specific polyadenylation signal sequences.

Preferably, the cell system used for the expression of the expression vector of the invention is selected from eukaryotic systems, for example insect cells such as SF-9, SF-21, High Five, Mimic, Drosophila S2, yeast cells, for example *S. cerevisiae* and *Pichia pastoris,* mammalian cells such as CHO and BHK cells, protozoan parasite cells such as *Leishmania tarentolae.* Alternatively, the cellular expression system may be a prokaryotic system, for example *E. coli* bacterial cells.

A pharmaceutical composition comprising the peptide as defined in appended claims 1 or 2, or the expression vector as defined in appended claims 3 or 4, or the host cell as defined in appended claim 5, for use in the therapeutic treatment of a neurodegenerative and/or neuro-traumatic disease, in combination with pharmaceutically acceptable carriers, excipients and/or diluents, is also within the scope of the invention.

The pharmaceutical composition for use according to the invention can be formulated into any suitable form, for example for enteral (oral or gastro-enteral, rectal, sublingual), parenteral (percutaneous, inhalation, ocular, intravenous, intra-arterial, intramuscular, intradermal, intranasal, subcutaneous, intraperitoneal) and topical administration (direct contact of the drug with the site of action and/or the skin and/or the mucous membranes and/or the ocular surface). Of course, the selection of suitable carriers, excipients and/or diluents is carried out depending on the desired form of administration and this selection is within the skills of those of ordinary skill in the art.

The experimental section that follows is provided for illustration purposes only and does not limit the scope of the invention as defined in the appended claims. In the experimental section, reference is made to the accompanying drawings, wherein:
Figure 1A shows the nucleotide sequences of the primers used for sequencing the cDNA coding for hproNGF-A. Figure 1B shows the nucleotide sequence of the cDNA coding for hproNGF-A, divided into the 3 exons that compose it. The non-coding regions (UTR) and the strictly coding region from the ATG start codon are highlighted. Figure 1C shows the schematic structure of the hproNGF-A protein and its amino acid sequence.
Figure 2 shows the results of the comparative analysis between proNGF-A and mature NGF (mNGF) as regards (A) stability and (B) the effects on the central regulation of the sympathetic activity. (A) Representative photomicrograph of the results from the Western blot analysis performed on conditioned culture media of rat PC 12 cells stimulated with hproNGF-A (upper panel) or mNGF (lower panel) for 24 hours. The samples examined were collected at time 0 and 4, 8 and 24 hours post-stimulation. (B) Representative photomicrographs of the immunolocalization of the tyrosine hydroxylase enzyme on brain tissue (locus coeruleus) sections taken from rats after intranasal administration of proNGFA or mNGF. The analysis reveals greater arborization (sprouting) of noradrenergic nerve fibres in brain tissue from mNGF-treated rats compared to hproNGF-A-treated animals (ctr = controls).
Figure 3A shows a series of photomicrographs indicative of PC12 cell differentiation after exposure to serum-free medium (first picture from the left), mature NGF (mNGF, second picture from the left), hproNGF-A (third picture from the left), and hproNGF-B (fourth picture from the left). Figure 3B is a histogram showing the results from the viability test performed on PC12 cells in suspension after addition of the factors listed under A above.
Figure 4 illustrates the effect of the hproNGF-A or hproNGF-B treatment on the number of hippocampal cells in healthy and diabetic rats. Figure 4A shows a representative photomicrograph of cells labelled with the Hoechst DNA intercalator. The broken lines show regions of the Dentate Gyrus (DG-H and DG-Granule Layer) where cell counts were performed, shown in the graphs in Figure 4B. Figure 4B shows four series of photomicrographs and underlying related histograms representative of the results from the quantitative serological analysis performed on the total cell number in healthy rats (upper part of panel B) and diabetic rats (lower part of panel B) in the DG-H (left) and DG-granule layer (right), respectively. Mean ± SEM, n=8 fields, 4 animals per experimental group, one way ANOVA followed by post-hoc Bonferroni test (P values calculated in comparison with corresponding saline-treated animals are shown in the figure).

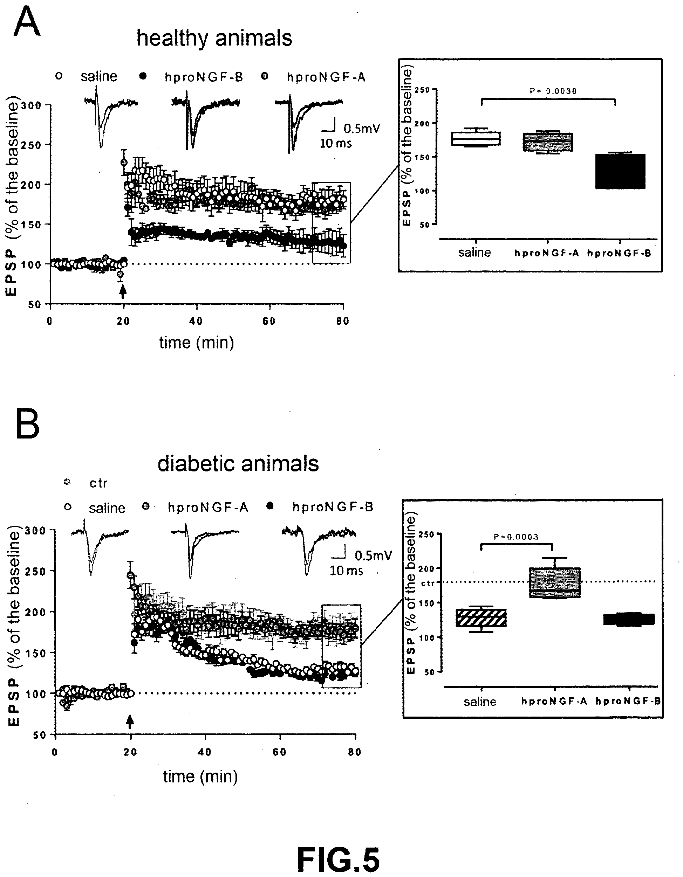

Figure 5 shows the effect of the hproNGF-A or hproNGF-B treatment on hippocampal synaptic plasticity. The left side of Figure 5 shows two graphs representing the results of the Long Term Potentiation (LTP) performed in bath perfusion on the Dentate Gyrus of healthy (A) and diabetic (B) rats, expressed as a percentage of the pre-tetanus baseline prior to LTP induction and 60 minutes after LTP induction by high frequency stimulation (HFS) of the medial perforant pathway (Mean ± SEM, n=8 animals per experimental group). The right side of Figure 5 shows two inserts corresponding to the aforementioned graphs, respectively, which illustrate box-plot graphs of the increase in the DG-LTP 50-60 minutes after HFS. Median ± interquartile. One-way ANOVA followed by post-hoc Bonferroni test (n = 8, P values calculated in comparison with corresponding saline-treated animals are shown in the figure).

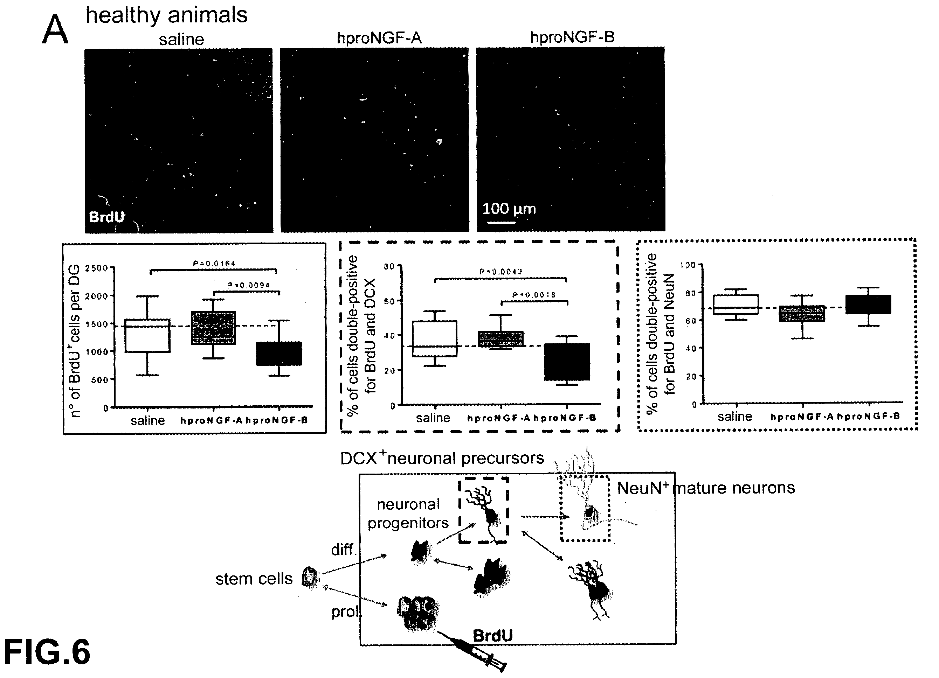

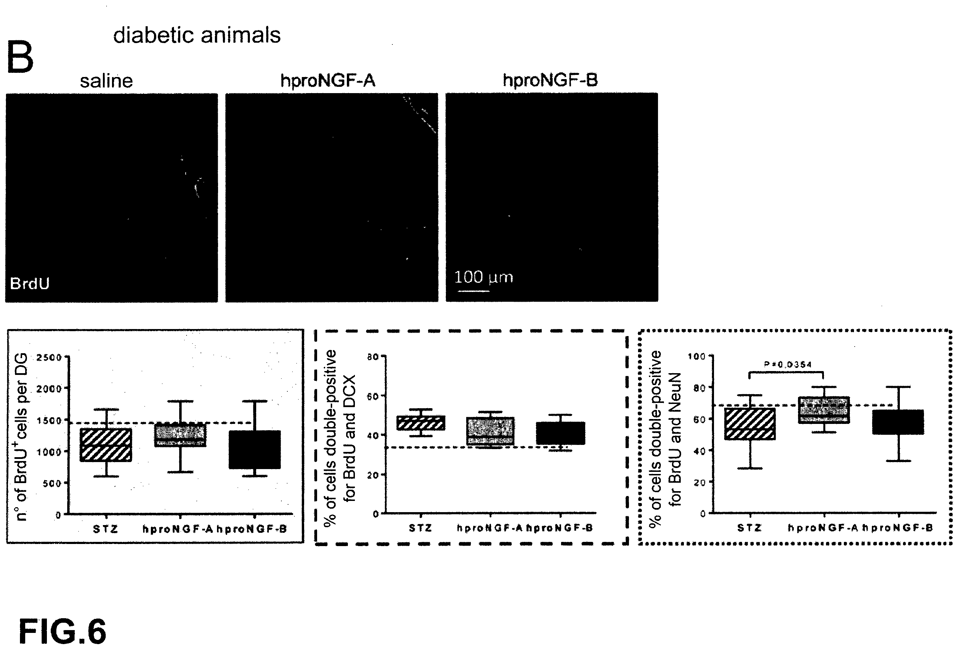

Figure 6 illustrates the effect of the hproNGF-A or hproNGF-B treatment on hippocampal neurogenesis in healthy rats (A) and diabetic rats (B). The upper part of Figure 6A and 6B shows a series of photomicrographs representative of healthy (A) and diabetic (B) rats treated with hproNGF-A or proNGF-B. In the central part of Figure 6A and 6B, the box-plot graphs enclosed by a solid line box represent the results from the quantitative analyses in healthy rats (A) and diabetic rats (B) of total cells that have incorporated BrdU, as shown in the diagram below Figure 6A, which describes the different cell types that have been included in the analyses reported in the different graphs. The box-plot graphs enclosed by a broken line show the results from the quantitative analyses in healthy rats (A) and diabetic rats (B) of cells that have incorporated BrdU and are at the same time positive for Doublecortin (DCX) (graphs enclosed by a dashed line box) or NeuN (graphs enclosed by a dotted line box). Median ± interquartile, n=4 animals per experimental group. One-way ANOVA followed by post-hoc Bonferroni test (the P values are shown in the figure).

### EXAMPLE 1: Isolation and sequencing of the sequence encoding the hproNGF-A peptide

In order to isolate the sequence encoding the peptide for use according to the invention, a PCR reaction was set up by using a pair of amplification primers having the sequences shown below:

| | |
|---|---|
| *Forward:* | TGGCCTCATCTAATGGACACT (SEQ ID NO:4) |
| *Reverse:* | GAAAGCTGCTCCCTTGGTAA (SEQ ID NO:5) |

The above primers were designed so that the PCR assay could discriminate the tissue expression of the putative transcript encoding the peptide for use according to the invention, designated as hproNGF-A, from the transcript already described in the literature encoding the hproNGF-B isoform. The sequences of the primer pair used for selective amplification of the hproNGF-B variant are as follows:

| | |
|---|---|
| *Forward:* | AGAGAGCGCTGGGAGCCGGA (SEQ ID NO:6) |
| *Reverse:* | GGACGCTGAGCTGAGCTTGGG (SEQ ID NO:7) |

The results of the PCR analysis performed on cDNA samples reverse transcribed from mRNA obtained from human brain and synovial tissue biopsies showed the presence of the transcript encoding for the new proNGF variant, designated as hproNGF-A, in various human tissues, both in the central nervous system and peripheral tissues.

The cDNA corresponding to the hproNGF-A transcript isolated from human brain biopsies was sequenced by the Sanger 5'-, internal-reverse- and 3'-UTR method (Eurofins Genomics) and its structure is shown in Figure 1.

### EXAMPLE 2: Construction of a Baculovirus expression system for the manufacture of

### the hproNGF-A peptide

The cDNA samples obtained from human brain tissue were amplified by PCR using a pair of specific primers (cloning primers) designed as follows:

| | |
|---|---|
| *Forward:* | ATGGCCTCATCTAATGGACA (SEQ ID NO:8) |
| *Reverse:* | GGCTCTTCTCACAGCCTT (SEQ ID NO:9) |

The amplification product was then inserted in a specific linearised plasmid vector, pFastBac ^{™} /CT-TOPO^{®}, containing Vaccinia virus DNA topoisomerase I covalently bound to the 3' end of each DNA strand (referred to as "TOPO^{®} activated" vector) using the Bac-to-Bac^{®} C.HIS TOPO^{®} Cloning Kit marketed by Thermo Scientific (*Catalog Number*: A11098) following the instructions supplied with the product. Insertion of the coding fragment in the plasmid was obtained in a test tube by blunt-end recombination catalysed by the topoisomerase enzyme. The above-described reaction provided a plasmid encoding the peptide for use according to the invention, to which a "tail" (tag) of six residues of the histidine amino acid (6xHis) was added, which is required during purification of the recombinant protein.

The reaction product was then used to transform by the heat-shock method chemically competent *One Shot*^{®} *Mach1*^{™} *T1R E. coli* bacteria, used for the multiplication of the plasmid vector, following the instructions contained in the *Bac-to-Bac*^{®} *C-HIS TOPO*^{®} *Cloning Kit (Catalog number: A11098, Thermo Scientific).* The transformed bacteria were then seeded on Petri dishes containing solid agarose medium with addition of ampicillin, required for the selection of recombinant bacteria, which through the plasmid have also incorporated the gene for resistance to this antibiotic. After the time required for bacterial growth (overnight at 37°C), ampicillin-resistant colonies were selected, picked, and used for plasmid DNA isolation by using the *PureLink*^{®} *HiPure Mini Plasmid Purification Kit (Catalog Number: K210002 Thermo Scientific),* following the instructions in the kit. The correctness of the direction of insertion was assessed by sequencing the expression clones with the primers supplied in the cloning kit, according to the instructions supplied with said kit. The purified plasmid was used to transform, by heat shock, competent *Max Efficiency*^{®} *DH10Bac*^{™} bacterial cells provided with the *Bac-to-Bac*^{®} *TOPO*^{®} *Expression System* kit *(Catalog Number: A11100, Thermo Fisher).* These cells, through proteins encoded by a helper plasmid contained therein, recombine by incorporating the pFastBac ^{™} /CT-TOPO^{®} plasmid into a larger plasmid, called bacmid, which will subsequently be incorporated by transfection into insect cells (SF-9), thus making them able to produce a recombinant baculovirus containing the hproNGF-A encoding sequence. After the recombination reaction, the bacterial cells were cultured in liquid LB-AGAR medium according to the instructions supplied with the kit, and then the bacmid produced by them was purified by using the *PureLink*^{™} *HiPure Plasmid Maxiprep Kit (Catalog Number: K210007, Thermo Scientific)* following the instructions therein. The presence and correct orientation of the DNA sequence coding for the peptide for use according to the invention was assessed by PCR with specific sequencing primers included in the *Bac-to-Bac*^{®} *TOPO*^{®} *Expression System* kit (*Catalog Number: A11100 Thermo Fisher*).

### EXAMPLE 3: Production of the hproNGF-A peptide in SF-9 cells

The bacmid obtained as described in Example 2 was used to transfect, through the Cellfectin^{®} II reagent (*Catalog Number: 10362100, Thermo Scientific*), SF-9 insect cells cultured in serum-free medium Sf-900 II SFM (*Catalog Number: 10902088, Thermo Scientific).*

The methodology indicated in the protocol for the use of the Cellfectin^{®} II reagent (*Catalog Number: 10362100, Thermo Scientific)* and in the *Bac-to-Bac*^{®} *TOPO*^{®} *Expression System (Catalog Number: A11100, Thermo Fisher)* was followed for this purpose. About 72 hours after transfection, the cell culture medium containing the recombinant baculovirus generated by the cells was clarified by centrifugation and plaque assayed, as described in the instructions of the *Bac-to-Bac*^{®} *TOPO*^{®} *Expression System* kit (*Catalog Number: A11100, Thermo Fisher*), to verify the infective titre of the virus produced. After verifying that the virus had a minimum titre of 10⁷ CFU, this first viral stock (P1) was used to infect a larger amount of SF-9 cells which, in a manner similar to that described above, produced a second viral stock (P2) and simultaneously expressed the recombinant hproNGF-A peptide, which was released into the culture medium and purified as described below.

### EXAMPLE 4: Purification of the hproNGF-A peptide

In order to purify the peptide for use according to the invention, the culture medium was centrifuged at 10000g for 45 minutes and filtered with 0.2-micron low-protein-retention filters (Millex GP, Millipore), to remove the virus particles and cell fragments, and was contacted with a Ni-Sepharose resin (GE Healthcare), which selectively binds the 6xHis fragments at the C-terminus of the recombinant protein. The resin, equilibrated with 20 mM phosphate buffer + 0.5M NaCl + 30 mM Imidazole (binding buffer), and the culture medium were mixed in a ratio of 1:5 v/v and subjected to orbital shaking at 200 rpm overnight at +4°C, to allow the 6xHIS residues to bind to the activated groups on the resin. After the binding, the resin was packed into a chromatography column (XK 16/20, GE Healthcare) and washed with 5 column volumes of the binding buffer. The protein was then eluted by subjecting the column to a 30 mM - 500 mM imidazole gradient in 20 column volumes. The eluted protein was dialysed against ultrapure water for 24 hours, then the dialysed material was frozen and freeze-dried.

After the freeze-drying, the protein was resuspended at a concentration of 1 mg/ml in an appropriate buffer (50 mM Tris-HCl pH 7.5 + 150 mM NaCl + 0.5 mM EDTA + 1 mM DTT) and subsequently the Tobacco Etch Virus (TEV) protease (Catalog Number T4455, Sigma Aldrich) was added in a ratio of 100U protease per milligram of protein. The TEV protease-mediated reaction is used to remove the 6xHIS tail (tag) linked to the C-terminus of the protein through a short amino acid bridge containing the site recognized by the TEV protease. For the enzymatic cleavage reaction, the reaction mixture was incubated at 30°C for two hours and then dialysed overnight against 20 mM phosphate buffer + 0.5 M NaCl + 30 mM Imidazole (binding buffer). The dialysate was then loaded onto a Ni-Sepharose resin (GE Healthcare), which selectively binds the 6xHis fragments at the N-terminus of the recombinant TEV protein. The resin, equilibrated with 20 mM phosphate buffer + 0.5M NaCl + 30 mM Imidazole (binding buffer), and the dialysate were mixed in a ratio of 1:5 v/v and subjected to orbital shaking at 200 rpm for 4 hours at +4°C, to allow the TEV 6xHis residues to bind to the activated groups on the resin. After the binding, the resin was packed into a chromatography column (PD10, GE Healthcare) and washed with 5 column volumes of the binding buffer. Human recombinant hproNGF-A from which the C-terminal 6xHis fragments had been deleted is present in this wash. The eluted protein was dialysed against ultrapure water for 24 hours, thereafter the dialysed material was frozen and freeze-dried.

The lyophilisate was resuspended overnight in 25 mM Sodium Acetate pH 5.0 + 0.2 M NaCl. The dialysate was then loaded onto a SP-Sepharose FF 16/10 column (GE Healthcare) to further purify hproNGF-A by cation exchange chromatography. After loading the column and washing with 10 column volumes of 25 mM sodium acetate buffer pH 5.0 + 0.2 M NaCl, hproNGF-A was eluted with a 0.2-1 M NaCl gradient in 20 column volumes. The chromatographic peak corresponding to hproNGF-A was collected, dialysed for 24 hours against 20 litres of ultrapure water and subsequently frozen and freeze-dried.

### EXAMPLE 5: SDS-PAGE, Coomassie and Western-blot assays

The purity and specificity of the purified protein was assayed by SDS-PAGE, Coomassie staining and Western blot.

In short, a total of 1 µg of purified protein was treated with 4X loading buffer (62.5 mM Tris HCl pH 6.8, 20% (v/v) glycerol, 8% (w/v) SDS, 0.025% (w/v) bromphenol blue and 100 mM dithiothreitol) and heated to 90°C for 5 minutes. The samples were resolved by SDS-PAGE electrophoresis in a 8-12% gradient polyacrylamide gel at 25-30 mA in running buffer (25 mM Tris HCl, 190 mM glycine adjusted to pH 8.3 and 0.1% (v/v) SDS). After the electrophoresis, the gel was subjected to Coomassie staining or the proteins were transferred onto a nitrocellulose membrane overnight at 30 V in transfer buffer (25 mM Tris-HCl, 190 mM glycine adjusted to pH 8.3 and 20% (v/v) methanol) to perform the Western blot. The membranes on which the proteins were transferred from the polyacrylamide gel were rinsed in PBS + 1% Tween 20 (T-PBS), blocked in T-PBS containing 5% non-fat freeze-dried milk for 1 hour at room temperature and then incubated overnight with appropriate anti-proNGF primary antibodies at 4°C. The membranes were then extensively washed in T-PBS at room temperature and incubated with horseradish peroxidase (HRP)-conjugated secondary antibody. After the incubation, the binding of the secondary antibody to the primary antibody was detected with the enhanced chemiluminescence system (*cod WBKLS0500, Millipore*).

### EXAMPLE 6: Comparative analysis between the hproNGF-A peptide and mature NGF

In order to assess the resistance of hproNGF-A and mature NGF to the action of extracellular proteases, rat pheochromocytoma cells (PC12) were stimulated with either of the two peptides. Samples collected from conditioned cell culture media at successive times (time zero; 4, 8 and 24 hours post-stimulation) were subjected to Western blot assay in order to verify the permanence of hproNGF-A and mNGF in the culture medium. The assay revealed that NGF remains in the culture medium for not more than 8 hours after the stimulus, (Figure 2A, lower panel), whereas proNGF-A is still present in the culture medium 24 hours after the stimulus (Figure 2A, upper panel). These results clearly demonstrate the significant lower susceptibility of the proNGF-A peptide to the action of extracellular proteases compared to the mNGF peptide.

The present inventors also carried out experiments aimed at testing the effects of the action of NGF and the proNGF-A peptide on the central regulation of the neuronal sympathetic activity. In short, the NGF or proNGF-A peptides were administered intranasally to the brain parenchyma of adult rats. Subsequently, the noradrenergic neurons located in the locus coeruleus, a brain region responsible for the central control of the functionality of the sympathetic nervous system, were analysed by immunolabelling on thin slices for tyrosine hydroxylase enzyme. Tyrosine hydroxylase enzyme represents an indirect measurement of the production of the noradrenaline neurotransmitter in neurons of certain brain areas. The expression of the tyrosine hydroxylase gene is stimulated by NGF and is responsible for some of the side effects described as a result of the administration of this peptide via the systemic route. The results of the immunolocalization assay illustrated in Figure 2B show that NGF administration causes arborization (sprouting) of the noradrenergic fibres, which contain tyrosine hydroxylase, in the locus coeruleus. This nucleus, located at the back of the brain, produces noradrenaline following the administration of the NGF peptide to the brain parenchyma and activates other brain nuclei (hypothalamus, thalamus), thereby generating an excessive response to stressful events as well as an increased sensitivity to painful stimuli. The presence of a greater number of fibres containing tyrosine hydroxylase (and thus producing noradrenaline) after NGF administration is indicative of the onset of side effects related to activation of the sympathetic nervous system (stress, hypothalamus) and increased sensitivity to pain (thalamus). Following *in vivo* administration of proNGF-A, the sprouting of noradrenergic fibres from the locus coeruleus decreases considerably (Figure 2B), thereby indicating that this peptide causes significantly lower side effects, if any.

### EXAMPLE 7: In vitro assessment of the biological activity

In order to perform a comparative analysis between the biological activity of the peptide for use according to the invention and the activities of the mNGF peptide and the proNGF-B isoform, an *in vitro* test was performed by using PC12 cells, which differentiate into the neuronal phenotype upon contact with neurotrophic factors such as, for example, the mNGF peptide. PC12 cells were cultured in RPMI 1640 culture medium supplemented with 10% horse serum and 5% calf serum in a humidified atmosphere with 5% CO₂ at 37°C. The exponentially growing cells were then seeded on 6-well plates containing poly-L-Lysine, to allow adherence of the cells to the bottom of the well. The cells were washed three times to remove any traces of serum, maintained in serum-free culture medium for one hour and then exposed to serum-free medium containing 50 ng/ml mNGF or 100 ng/ml hproNGF-A or hproNGF-B. After a five-day incubation in the above-described culture conditions, only cells exposed to mNGF and hproNGF-A exhibited the characteristic extensions (neurites) that testify successful neuronal differentiation, whereas cells exposed to hproNGF-B showed evident signs of suffering, as attested by their small size, the tendency to detach from the bottom of the well, and the presence of numerous cellular debris in the culture medium (as shown in Figure 3A). Moreover, the viability test performed by exposing PC12 cells in suspension to the culture conditions described above showed that, while the hproNGF-B peptide induces a decrease in the percentage of living cells after 48 hours of culture, both the mNGF peptide and the hproNGF-A peptide for use according to the invention maintain a significant proportion of living cells after serum deprivation, testifying their neuroprotective action (as shown in the histogram in Figure 3B).

### EXAMPLE 8: In vivo assessment of the biological activity

An animal diabetes-induced neurodegeneration model in male Wistar strain rats weighing 250-300 grams was employed to assess the *in vivo* biological activity of the peptide for use according to the invention compared to the activity of the hproNGF-B peptide. The diabetes in rats was induced via a single intraperitoneal injection (i.p.) of 65 mg/kg streptozotocin *(Cat. S0130, Sigma-Aldrich, Milan, Italy),* dissolved in citrate buffer pH 4.5. After a period of one or four weeks of streptozotocin treatment, the onset of diabetes was assessed with an Accutrend^{™} GC glucose analyzer (Roche Diagnostics, Germany). Rats with blood glucose levels above 300 mg/dl were assigned to diabetic experimental groups. The animals were euthanized four weeks after diabetes was induced.

Within the experimental design described above and four days prior to euthanasia, control and diabetic rats were intranasally administered once a day for four days with purified hproNGF-A peptide (n = 12 rats), hproNGF-B peptide (n = 12 rats) or phosphate buffered saline (PBS) as the vehicle (n = 12 rats). Intranasal administration is the chosen method for the administration of therapeutic agents to the central nervous system because it is not invasive and allows the problems related to the crossing of the blood-brain barrier of the central nervous system (CNS) by large molecules to be overcome. In addition, drugs administered intranasally are directly targeted to the central nervous system, thus reducing systemic exposure and hence any unwanted systemic side effects. Intranasal administration is a widely accepted method and is currently used in human clinical trials.

To check whether the opposite nature of the two different hproNGF isoforms could affect the number of hippocampal cells and whether the intranasal administration of proNGF could have an effect on diabetes-induced neuronal loss, Hoechst-positive cells were analysed by confocal microscopy in both the cell layers of the hippocampal dentate gyrus, the granule layer (GL) and the hilus (HL) (as shown in Figure 4A). The rats were deeply anesthetized and transcardially perfused with 0.1 M phosphate buffer followed by 4% paraformaldehyde fixative in 0.1 M phosphate buffer. The brains were removed and cryoprotected with 30% sucrose in phosphate buffer. 40 µm-thick coronal sections of both hemispheres, ranging - 2,40 / -3,72 mm from the bregma according to the Paxinos Rat Brain Atlas, were cut on a cryostat and then processed. The brain sections were pre-incubated for 2 hours at room temperature (RT) with PBS containing 10% donkey serum (v/v), 1% BSA (w/v) and 0.3% Triton X-100 (v/v). After washing with PBS, the sections were incubated (2 h, RT) with specific secondary antibodies. Subsequently, the sections were rinsed three times in PBS and incubated for 10 minutes with Hoechst dye for specific staining of the nuclei. Both in the hilus and the granule layer, control rats treated with the hproNGF-A peptide for use according to the invention showed a number of cells similar to that observed in saline-treated rats, whereas the number of cells decreased significantly in rats treated with the hproNGF-B peptide compared to saline-treated animals (as shown in Figure 4B, upper panel). The results of the *in vivo* analysis described above are consistent with the *in vitro* findings illustrated in the paragraph of Example 7, according to which the two peptide isoforms have fundamentally different effects on cell viability.

Since the negative effect of diabetes on Long-Term Potentiation (LTP), a neurophysiological correlate of hippocampal function, has long been known, it was further investigated whether the administration of the two distinct hproNGF isoforms modulated the hippocampal neural plasticity. For extracellular LTP recordings, hippocampal slices (350 µm thick) were kept immersed at 30°C and superfused (2-3 ml/min-1) with oxygenated cerebrospinal fluid (95% O2, 5% CO2) (ACSF; 126 mM NaCl , 2 mM KCl, 1 mM NaH2PO4, 24 mM NaHCO3, 2 mM CaCl2, 1 mM MgCl2, 10 mM glucose). Stimulation was applied to the medial perforant pathway of the dentate gyrus (DG) by using a bipolar insulated tungsten wire electrode and the field excitatory postsynaptic potentials were recorded at a control test frequency of 0.033 Hz from the middle one-third of the molecular layer of the DG with a glass microelectrode. Long-term potentiation (LTP) was evoked by high-frequency stimulation (HFS) consisting of eight pulse trains, each with eight stimuli at 200 Hz and an inter-train interval of 2 s, with the stimulation voltage increased during the HFS protocol. LTP measurements were made 60 minutes after HFS. All solutions contained 50 µM picrotoxin (P1675, Sigma-Aldrich) to block GABA-A-mediated activity and facilitate LTP.

As shown in Figure 5A, in the control groups (healthy animals), administration of the hproNGF-A peptide for use according to the invention did not affect the DG-LTP, which is comparable to that of the saline-treated group, whereas the extent of DG-LTP in animals treated with the hproNGF-B peptide is reduced by 28%, thus revealing damage induced by administration of hproNGF-B. In the groups of diabetic animals, administration of hproNGF-A improved the streptozotin-induced DG-LTP decrease by 38%, thereby restoring control levels. Instead, the present study showed that the DG-LTP in hproNGF-B-treated rats is not different from that measured in saline-treated diabetic rats (as shown in Figure 5B).

The experimental results described above demonstrate that treatment with the hproNGF-A peptide for use according to the invention is capable of improving the impaired functionality of the hippocampus in diabetic rats.

It is also known that diabetic encephalopathy impairs the hippocampal neurogenesis process. In order to assess the effects induced by exogenous administration of the two hproNGF peptide isoforms on hippocampal neurogenesis, the hproNGF-A peptide for use according to the invention and the known hproNGF-B peptide were administered to healthy and diabetic rats.

In order to assess the effects of the treatments on dentate gyrus neurogenesis, at the time of diabetes induction, rats were inoculated with bromodeoxyuridine (BrdU), which intercalates into newly formed DNA, specifically labelling cells proliferating at the time of inoculation. Subsequently, a confocal microscopy analysis was performed by labelling with anti-BrdU antibodies and specific antibodies to detect the presence of immature neurons (antibodies to doublecortin, DCX) or mature neurons (antibodies to NeuN).

Microscopy analysis on samples from healthy rats revealed no effect of the hproNGF-A peptide for use according to the invention on the number of BrdU-immunopositive cells. In contrast, as shown in Figure 6A, a significant reduction in the number of cells that have incorporated BrdU was detected after treatment with hproNGF-B. In the same healthy animals, intranasal hproNGF-A administration had no significant effect on the percentage of cells simultaneously immunopositive for BrdU and DCX. Instead, treatment with the hproNGF-B peptide induced a significant reduction in the percentage of DCX-immunopositive cells (as shown by the box-plot graph in Figure 6A, dashed line box).

The results described above show the negative effect of the hproNGF-B peptide isoform on newly generated cells.

In diabetic animals, no influence on the diabetes-induced alteration in the number of BrdU+-cells (as shown by the box-plot graph in Figure 6B, solid line box), or on the percentage of BrdU+-cells expressing DCX (as shown by the box-plot graph in Figure 6B, dashed line box) was found as a result of treatments with the hproNGF-A and hproNGF-B peptides. However, a significant increase in the percentage of BrdU-immunopositive cells that were also NeuN-immunopositive (as shown by the box-plot graph in Figure 6B, dotted line box) was found in diabetic rats treated with the hproNGF-A peptide for use according to the invention. This finding indicates that the hproNGF-A peptide variant, the object of the invention, has a potential neuroprotective role in neurodegenerative diseases characterised by decreased hippocampal neurogenesis, such as, for example, diabetic encephalopathy and Alzheimer's disease, by promoting survival and terminal differentiation of hippocampal stem cells into mature neurons.

## Claims

1. An isolated peptide consisting of the amino acid sequence SEQ ID NO: 1, for use in the therapeutic treatment of a neurodegenerative disease.

2. The peptide for use according to claim 1, which is encoded by nucleic acid sequence SEQ ID NO:3.

3. An expression vector for use in the therapeutic treatment of a neurodegenerative disease, said expression vector comprising a nucleic acid sequence encoding the peptide according to claim 1.

4. The expression vector for use according to claim 3, comprising SEQ ID NO:3 or SEQ ID NO: 2.

5. A host cell for use in the therapeutic treatment of a neurodegenerative disease, said host cell comprising an expression vector according to claim 3 or 4.

6. The peptide for use according to claim 1 or 2, or the expression vector for use according to claim 3 or 4, or the host cell for use according to claim 5, wherein the disease is an acute or chronic neurodegenerative disease selected from the group consisting of ischemic or hemorrhagic stroke, traumatic brain injury, intracranial hypertension, cerebral edema, perinatal, pediatric or adult hypoxia/ischemia, hypoxia/ischemia caused by cardiac arrest, drowning or hypothermia, Alzheimer's disease, Parkinson's disease, Huntington's Chorea, amyotrophic lateral sclerosis (ALS), progressive supranuclear palsy, frontotemporal dementia, Lewy body dementia, Creutzfeldt-Jakob disease (CJD), Gerstmann-Sträussler-Scheinker disease (GSS).

7. A pharmaceutical composition for use in the therapeutic treatment of a neurodegenerative disease, said composition comprising a peptide according to claim 1 or 2, or an expression vector according to claim 3 or 4, or a host cell according to claim 5, and at least one pharmaceutically acceptable carrier, excipient and/or diluent.

8. The pharmaceutical composition for use according to claim 7, which is in a form suitable for enteral, parenteral or topical administration.

## Patentansprüche

1. Isoliertes Peptid, bestehend aus der Aminosäuresequenz SEQ ID Nr. 1, zur Verwendung in der therapeutischen Behandlung einer neurodegenerativen Erkrankung.

2. Peptid zur Verwendung nach Anspruch 1, welches durch die Nukleinsäuresequenz SEQ ID Nr. 3 kodiert ist.

3. Expressionsvektor zur Verwendung in der therapeutischen Behandlung einer neurodegenerativen Erkrankung, wobei der Expressionsvektor eine Nukleinsäuresequenz aufweist, die das Peptid nach Anspruch 1 kodiert.

4. Expressionsvektor zur Verwendung nach Anspruch 3, aufweisend SEQ ID Nr. 3 oder SEQ ID Nr. 2.

5. Wirtszelle zur Verwendung in der therapeutischen Behandlung einer neurodegenerativen Erkrankung, wobei die Wirtszelle einen Expressionsvektor nach Anspruch 3 oder 4 aufweist.

6. Das Peptid zur Verwendung nach Anspruch 1 oder 2, oder der Expressionsvektor zur Verwendung nach Anspruch 3 oder 4, oder die Wirtszelle zur Verwendung nach Anspruch 5, wobei die Erkrankung eine akute oder chronische neurodegenerative Erkrankung ausgewählt aus der Gruppe bestehend aus ischämischem oder hämorrhagischem Schlaganfall, traumatischer Hirnverletzung, intrakranieller Hypertonie, Hirnödem, Hypoxie/Ischämie, vorgeburtlich, bei Kindern oder Erwachsenen, Hypoxie/Ischämie durch Herzstillstand, Ertrinken oder Unterkühlung, Alzheimer-Krankheit, Parkinson-Krankheit, Chorea Huntington, amyotropher Lateralsklerose (ALS), progressiver supranukleärer Lähmung, frontotemporaler Demenz, Lewy-Körper-Demenz, Creutzfeldt-Jakob-Krankheit (CJD), Gerstmann-Sträussler-Scheinker-Krankheit (GSS) ist.

7. Pharmazeutische Zusammensetzung zur Verwendung in der therapeutischen Behandlung einer neurodegenerativen Erkrankung, wobei die Zusammensetzung ein Peptid nach Anspruch 1 oder 2 oder einen Expressionsvektor nach Anspruch 3 oder 4 oder eine Wirtszelle nach Anspruch 5 und mindestens einen pharmazeutisch zulässigen Träger, Hilfsstoff und/oder ein pharmazeutisch zulässiges Verdünnungsmittel aufweist.

8. Pharmazeutische Zusammensetzung zur Verwendung nach Anspruch 7, welche in einer zur enteralen, parenteralen oder lokalen Verabreichung geeigneten Form vorliegt.

## Revendications

1. Peptide isolé se composant de la séquence d'acides aminés SEQ ID NO : 1, pour utilisation dans le traitement thérapeutique d'une maladie neurodégénérative.

2. Peptide pour utilisation selon la revendication 1, qui est codé par une séquence d'acides nucléiques SEQ ID NO : 3.

3. Vecteur d'expression pour utilisation dans le traitement thérapeutique d'une maladie neurodégénérative, ledit vecteur d'expression comprenant une séquence d'acides nucléiques codant pour le peptide selon la revendication 1.

4. Vecteur d'expression pour utilisation selon la revendication 3, comprenant le SEQ ID NO : 3 ou le SEQ ID NO : 2.

5. Cellule hôte pour utilisation dans le traitement thérapeutique d'une maladie neurodégénérative, ladite cellule hôte comprenant un vecteur d'expression selon la revendication 3 ou 4.

6. Peptide pour utilisation selon la revendication 1 ou 2, ou vecteur d'expression pour utilisation selon la revendication 3 ou 4, ou cellule hôte pour utilisation selon la revendication 5, dans lequel la maladie est une maladie neurodégénérative aiguë ou chronique sélectionnée dans le groupe composé d'un accident vasculaire cérébral ischémique ou hémorragique, d'un traumatisme crânien, d'une hypertension intracrânienne, d'un oedème cérébral, d'une hypoxie/ischémie périnatale, pédiatrique ou adulte, d'une hypoxie/ischémie provoquée par un arrêt cardiaque, de la noyade ou de l'hypothermie, de la maladie d'Alzheimer, de la maladie de Parkinson, de la chorée de Huntington, d'une sclérose latérale amyotrophique (SLA), d'une paralysie supranucléaire progressive, de la démence frontotemporale, de la démence à corps de Lewy, de la maladie de Creutzfeldt-Jakob (MCJ), du syndrome de Gerstmann-Straüssler-Scheinker (GSS).

7. Composition pharmaceutique pour utilisation dans le traitement thérapeutique d'une maladie neurodégénérative, ladite composition comprenant un peptide selon la revendication 1 ou 2, ou un vecteur d'expression selon la revendication 3 ou 4, ou une cellule hôte selon la revendication 5, et au moins un support, un excipient et/ou un diluant pharmaceutiquement acceptables.

8. Composition pharmaceutique pour utilisation selon la revendication 7, qui se présente sous une forme appropriée pour une administration entérale, parentérale ou topique.
